(19) Europäisches Patentamt
European Patent Office
Office européen des brevets

(11) Veröffentlichungsnummer: **0 257 422**
**A1**

(12) # EUROPÄISCHE PATENTANMELDUNG

(21) Anmeldenummer: **87111538.2**

(22) Anmeldetag: **10.08.87**

(51) Int. Cl.⁴: **C07C 79/46** , C07C 149/273 , C07C 147/00 , A01N 37/48 , A01N 41/10

(30) Priorität: **21.08.86 DE 3628317**

(43) Veröffentlichungstag der Anmeldung: **02.03.88 Patentblatt 88/09**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Förster, Heinz, Dr.**
**Am Eckbusch 47**
**D-5600 Wuppertal 1(DE)**
Erfinder: **Pfister, Theodor, Dr.**
**Lichtenbergerstrasse 30**
**D-4019 Monheim(DE)**
Erfinder: **Santel, Hans-Joachim, Dr.**
**Grünstrasse 9 a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Robert Harry, Dr.**
**Unterdorfstrasse 6A**
**D-4000 Düsseldorf 31(DE)**

(54) **Phenoxybenzoesäureester.**

(57) Die Erfindung betrifft neue Phenoxybenzoesäureester der Formel (I)

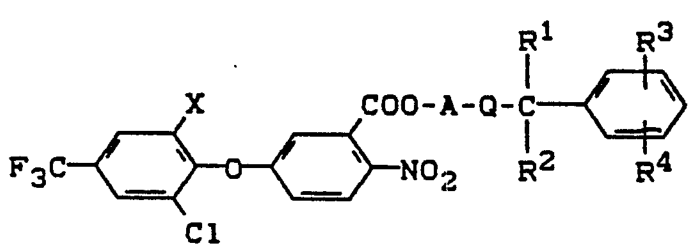

(I)

in welcher
X für Wasserstoff oder Halogen steht,
A für gegebenenfalls verzweigtes $C_2$-$C_6$-Alkandiyl steht,
Q für Sauerstoff, Schwefel, Sulfinyl (SO) oder Sulfonyl ($SO_2$) steht,
$R^1$ für Wasserstoff oder Methyl steht,
$R^2$ für Wasserstoff oder $C_1$-$C_4$-Alkyl steht und
$R^3$ und $R^4$ gleich oder verschieden sind und für Wasserstoff, Halogen, Cyano, Nitro, $C_1$-$C_4$-Alkyl, Halogen-$C_1$-$C_2$-alkyl, $C_1$-$C_4$-Alkoxy, Halogen-$C_1$-$C_2$-alkoxy und $C_1$-$C_2$-Alkoxycarbonyl stehen,

EP 0 257 422 A1

Verfahren zu deren Herstellung sowie ihre Verwendung als Herbizide.

## Phenoxybenzoesäureester

Die Erfindung betrifft neue Phenoxybenzoesäureester, Verfahren zu deren Herstellung sowie ihre Verwendung als Herbizide.

Es ist bereits bekannt, daß zahlreiche Phenoxybenzoesäure-Derivate herbizide Eigenschaften besitzen (vgl. US-P 4 063 929). So kann zum Beispiel das Natriumsalz der 3-(2-Chlor-4-trifluormethyl-phenoxy)-6-nitro-benzoesäure zur Bekämpfung von Unkraut eingesetzt werden. Die Wirkung dieses Stoffes ist gut, jedoch werden bei geringen Aufwandmengen einige Unkräuter nicht immer voll erfaßt.

Es wurden nun neue Phenoxybenzoesäureester der Formel (I)

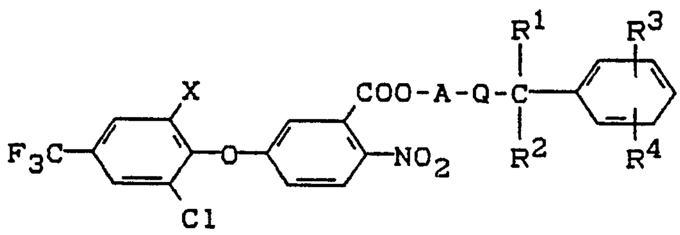

(I)

in welcher

X für Wasserstoff oder Halogen steht,

A für gegebenenfalls verzweigtes $C_2$-$C_6$-Alkandiyl steht,

Q für Sauerstoff, Schwefel, Sulfinyl (SO) oder Sulfonyl ($SO_2$) steht,

$R^1$ für Wasserstoff oder Methyl steht,

$R^2$ für Wasserstoff oder $C_1$-$C_4$-Alkyl steht und

$R^3$ und $R^4$ gleich oder verschieden sind und für Wasserstoff, Halogen, Cyano, Nitro, $C_1$-$C_4$-Alkyl, Halogen-$C_1$-$C_2$-alkyl, $C_1$-$C_4$-Alkoxy, Halogen-$C_1$-$C_2$-alkoxy und $C_1$-$C_2$-Alkoxycarbonyl stehen,

gefunden.

Für den Fall, daß A für verzweigtes $C_2$-$C_6$-Alkandiyl steht, enthalten die Phenoxybenzoesäureester der Formel (I) mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiterhin wurde gefunden, daß man Phenoxybenzoesäureester der Formel (I) erhält, wenn man

(a) Phenoxybenzoesäurechloride der Formel (II)

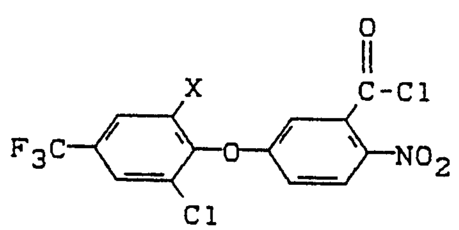

(II)

in welcher

X die oben angegebene Bedeutung hat,

mit Hydroxyverbindungen der Formel (III)

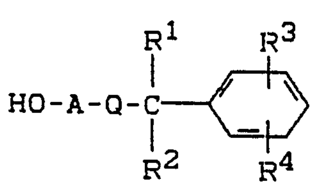

(III)

in welcher

A, Q, $R^1$, $R^2$, $R^3$ und $R^4$ die oben angegebenen Bedeutungen haben, gegebenenfalls in Gegenwart eines Säurebindemittels sowie gegebenenfalls in Gegenwart eines Verdünnungsmittels umsetzt,

oder wenn man außerdem für den Fall, daß Verbindungen der Formel (I) hergestellt werden sollen, in denen Q für Sulfinyl oder Sulfonyl steht,

(b) die Verbindungen der Formel (Ia)

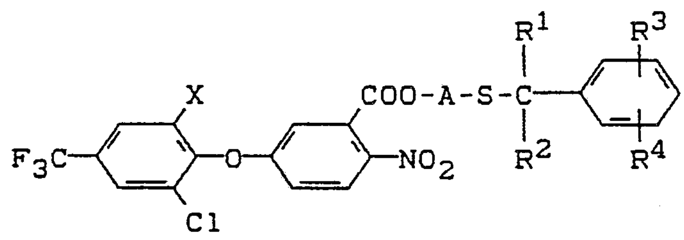

(Ia)

in welcher

A, X, $R^1$, $R^2$, $R^3$ und $R^4$ die oben angegebenen Bedeutungen haben, mit Oxidationsmitteln, gegebenenfalls in Gegenwart eines Katalysators und gegebenenfalls in Gegenwart eines Verdünnungsmittels umsetzt.

Schließlich wurde gefunden, daß sich die neuen Phenoxybenzoesäureester der Formel (I) durch eine hervorragende herbizide Wirkung auszeichnen.

Überraschenderweise sind die erfindungsgemäßen Phenoxybenzoesäureester der Formel (I) gegen einige wichtige Unkräuter wesentlich wirksamer und besitzen wesentlich bessere selektive Eigenschaften als das Natriumsalz der 3-(2-Chlor-4-trifluormethyl-phenoxy)-6-nitro-benzoesäure, welches ein strukturell ähnlicher, vorbekannter Wirkstoff analoger Wirkungsrichtung ist.

Die erfindungsgemäßen Phenoxybenzoesäureester sind durch die Formel (I) allgemein definiert. Bevorzugt sind diejenigen Verbindungen der Formel (I), in welchen

X für Wasserstoff, Fluor oder Chlor steht,

A für gegebenenfalls verzweigtes $C_2$-$C_5$-Alkandiyl steht,

Q für Sauerstoff, Schwefel, Sulfinyl oder Sulfonyl steht,

$R^1$ für Wasserstoff steht,

$R^2$ für Wasserstoff oder Methyl steht und

$R^3$ und $R^4$ gleich oder verschieden sind und für Wasserstoff, Fluor, Chlor, Cyano, Nitro, Methyl, Trifluormethyl, Methoxy, Trifluormethoxy oder Methoxycarbonyl stehen.

Besonders bevorzugt sind diejenigen Verbindungen der Formel (I), in denen

X für Wasserstoff oder Chlor steht,

A für gegebenenfalls verzweigtes $C_2$-$C_5$-Alkandiyl steht,

Q für Sauerstoff, Schwefel oder Sulfonyl steht,

$R^1$ für Wasserstoff steht,

$R^2$ für Wasserstoff oder Methyl steht und

$R^3$ und $R^4$ gleich oder verschieden sind und für Wasserstoff, Fluor, Chlor oder Methoxy stehen.

Verwendet man für das erfindungsgemäße Verfahren (a) 3-(2,6-Dichlor-4-trifluormethyl-phenoxy)-6-nitrobenzoesäurechlorid und 2-Benzyloxy-ethanol als Ausgangsstoffe, so kann der Verlauf des erfindungsgemäßen Verfahrens (a) durch das folgende Formelschema wiedergegeben werden:

a)

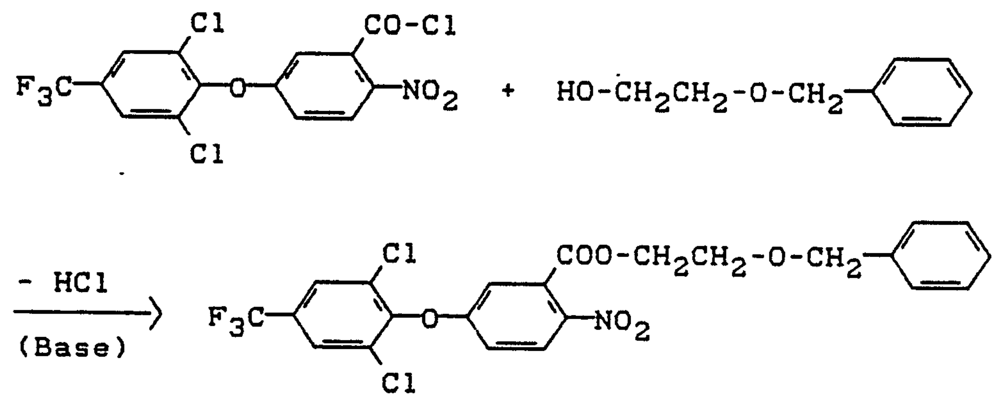


Verwendet man für das erfindungsgemäße Verfahren (b) 5-(2,6-Dichlor-4-trifluormethyl-phenoxy)-2-nitro-benzoesäure-(2-benzylthio-ethyl)-ester als Ausgangsstoff und Wasserstoffperoxid als Oxidationsmittel, so kann der Verlauf des erfindungsgemäßen Verfahrens (b) durch das folgende Formelschema wiedergegeben werden:

(b)

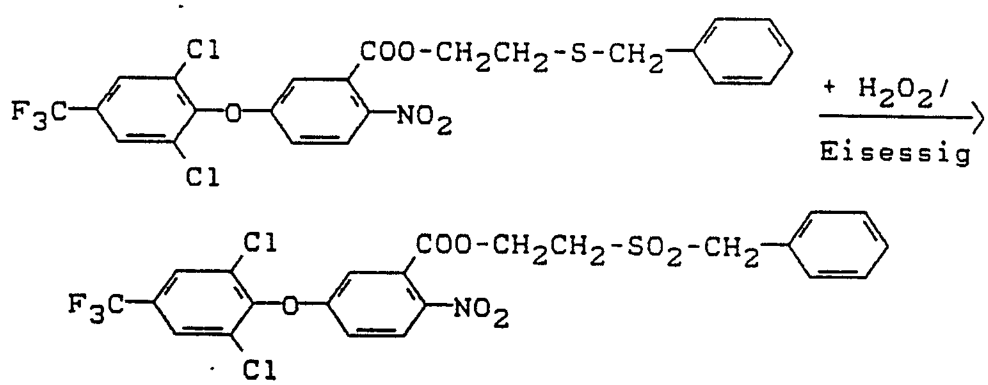


Die bei dem erfindungsgemäßen Verfahren (a) als Ausgangsstoffe benötigten Phenoxybenzoesäurechloride sind durch die Formel (II) definiert. In dieser Formel hat X vorzugsweise diejenigen Bedeutungen, die bereits im Zusammenhang mit der Beschreibung der erfindungsgemäßen Verbindungen der Formel (I) vorzugsweise für diesen Rest genannt wurden.

Als Beispiele für die Verbindungen der Formel (II) seien genannt:

3-(2-Chlor-4-trifluormethyl-phenoxy)-6-nitro-benzoesäurechlorid, 3-(2,6-Dichlor-4-trifluormethyl-phenoxy)-6-nitrobenzoesäurechlorid und 3-(2-Chlor-6-fluor-4-trifluormethyl-phenoxy)-6-nitro-benzoesäurechlorid.

Die Phenoxybenzoesäurechloride der Formel (II) sind bekannt oder lassen sich nach bekannten Verfahren in einfacher Weise herstellen (vgl. DE-OS 2 311 638, EP-OS 63 741 und EP-OS 122 037).

Die bei dem erfindungsgemäßen Verfahren (a) weiterhin als Ausgangsstoffe benötigten Hydroxyverbindungen sind durch die Formel (III) definiert. In dieser Formel haben A, Q, $R^1$, $R^2$, $R^3$ und $R^4$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (III) seien genannt:

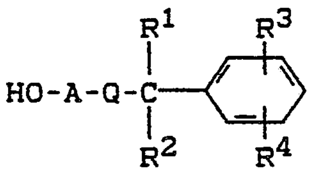

(III)

Tabelle 1

| A | Q | $R^1$ | $R^2$ | $R^3$ | $R^4$ |
|---|---|---|---|---|---|
| $-CH_2CH_2-$ | O | H | H | H | H |
| $-CH_2CH_2-$ | S | H | H | H | H |
| $-CH_2CH_2-$ | $SO_2$ | H | H | H | H |
| $-(CH_2)_3-$ | O | H | H | H | H |
| $-(CH_2)_3-$ | S | H | H | H | H |
| $-(CH_2)_3-$ | $SO_2$ | H | H | H | H |

<u>Tabelle 1</u> - Fortsetzung

| A | Q | $R^1$ | $R^2$ | $R^3$ | $R^4$ |
|---|---|---|---|---|---|
| $-CH-CH_2-$<br>\|<br>$CH_3$ | O | H | H | H | H |
| $-CH-CH_2-$<br>\|<br>$CH_3$ | S | H | H | H | H |
| $-CH-CH_2-$<br>\|<br>$CH_3$ | $SO_2$ | H | H | H | H |
| $CH_3$<br>\|<br>$-CH_2-C-CH_2-$<br>\|<br>$CH_3$ | O | H | H | H | H |
| $CH_3$<br>\|<br>$-CH_2-C-CH_2-$<br>\|<br>$CH_3$ | S | H | H | H | H |
| $CH_3$<br>\|<br>$-CH_2-C-CH_2-$<br>\|<br>$CH_3$ | $SO_2$ | H | H | H | H |
| $-CH_2CH_2-$ | O | H | H | 2-Cl | H |
| $-CH_2CH_2-$ | O | $CH_3$ | H | H | H |
| $-CH_2CH_2-$ | S | $CH_3$ | H | H | H |
| $-CH_2CH_2-$ | O | H | H | 2-Cl | 6-Cl |
| $-CH_2CH_2-$ | O | H | H | 4-F | H |
| $-CH_2CH_2-$ | S | H | H | 4-F | H |

Tabelle 1 - Fortsetzung

| A | Q | $R^1$ | $R^2$ | $R^3$ | $R^4$ |
|---|---|---|---|---|---|
| $-CH_2CH_2-$ | O | H | H | 4-Cl | H |
| $-CH_2CH_2-$ | S | H | H | 4-Cl | H |
| $-CH_2CH_2-$ | $SO_2$ | H | H | 4-Cl | H |
| $-CH_2CH_2-$ | O | H | H | $4-OCH_3$ | H |
| $-CH_2CH_2-$ | S | H | H | $4-OCH_3$ | H |
| $-CH_2CH_2-$ | O | H | H | 2-F | H |
| $-CH_2CH_2-$ | S | H | H | 2-F | H |
| $-CH_2CH_2-$ | O | H | H | 2-F | 6-F |
| $-CH_2CH_2-$ | S | H | H | 2-F | 6-F |
| $-CH_2CH_2-$ | O | H | H | $4-CH_3$ | H |

Die Verbindungen der Formel (III) sind bekannt oder lassen sich nach bekannten Verfahren in einfacher Weise herstellen (vgl. EP-OS 68 260 und EP-OS 92 112).

Das erfindungsgemäße Verfahren (a) zur Herstellung der neuen Phenoxybenzoesäureester der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z.B. Acetonitril und Propionitril Amide wie z.B. Dimethylformamid, Dimethylacetamid und N-Methyl-pyrrolidon sowie Dimethylsulfoxid und Tetramethylensulfon.

Als Säurebindemittel können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z.B. Natrium-und Kaliumhydroxid, Erdalkalihydroxide wie z.B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4,3,0]-nonen-5 (DBN) und 1,8-Diazabicyclo-[5,4,0]-undec-7-en (DBU).

Die Reaktionstemperaturen können bei dem erfindungsgemäßen Verfahren (a) zur Herstellung der Phenoxybenzoesäureester der Formel (I) in einem größeren Bereich variiert werden. Im allgemeinen arbeitet man bei Temperaturen zwischen -20 °C und +100 °C, vorzugsweise bei Temperaturen zwischen 0 °C und 80 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden.

Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) als Ausgangsstoffe zu verwendenden Verbindungen sind durch die Formel (Ia) allgemein definiert. In dieser Formel (Ia) stehen X, A, $R^1$, $R^2$, $R^3$ und $R^4$ vorzugsweise für diejenigen Reste, die bereits im Zusammenhang mit der Beschreibung der erfindungsgemäßen Stoffe der Formel (I) vorzugsweise für diese Substituenten genannt wurden.

Als Beispiele für die Verbindungen der Formel (Ia) seien genannt:

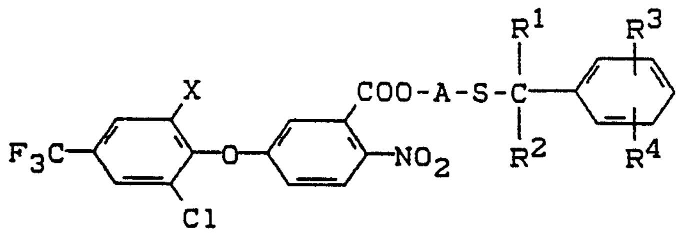

(Ia)

Tabelle 2

| X | A | $R^1$ | $R^2$ | $R^3$ | $R^4$ |
|---|---|---|---|---|---|
| H | $-CH_2CH_2-$ | H | H | H | H |
| Cl | $-CH_2CH_2-$ | H | H | H | H |
| H | $-(CH_2)_3-$ | H | H | H | H |
| Cl | $-(CH_2)_3-$ | H | H | H | H |
| H | $-CH(CH_3)-CH_2-$ | H | H | H | H |
| Cl | $-CH(CH_3)-CH_2-$ | H | H | H | H |

Tabelle 2 - Fortsetzung

| X | A | $R^1$ | $R^2$ | $R^3$ | $R^4$ |
|---|---|---|---|---|---|
| H | 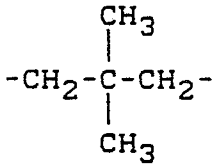 | H | H | H | H |
| Cl | 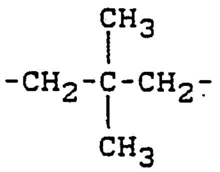 | H | H | H | H |
| H | $-CH_2CH_2-$ | $CH_3$ | H | H | H |
| Cl | $-CH_2CH_2-$ | $CH_3$ | H | H | H |
| H | $-CH_2CH_2-$ | H | H | 4-F | H |
| Cl | $-CH_2CH_2-$ | H | H | 4-F | H |
| H | $-CH_2CH_2-$ | H | H | 4-Cl | H |
| Cl | $-CH_2CH_2-$ | H | H | 4-Cl | H |
| H | $-CH_2CH_2-$ | H | H | $4-OCH_3$ | H |
| Cl | $-CH_2CH_2-$ | H | H | $4-OCH_3$ | H |
| H | $-CH_2CH_2-$ | H | H | 2-F | H |
| Cl | $-CH_2CH_2-$ | H | H | 2-F | H |
| H | $-CH_2CH_2-$ | H | H | 2-F | 6-F |
| Cl | $-CH_2CH_2-$ | H | H | 2-F | 6-F |
| H | $-CH_2CH_2-$ | H | H | $4-CH_3$ | H |
| Cl | $-CH_2CH_2-$ | H | H | $4-CH_3$ | H |

Die Verbindungen der Formel (Ia) sind erfindungsgemäße Verbindungen und werden gemäß Verfahren (a) hergestellt.

Als Oxidationsmittel können für das erfindungsgemäße Verfahren (b) alle üblichen sauerstoffabgebenden Oxidationsmittel eingesetzt werden. Vorzugsweise in Frage kommen Wasserstoffperoxid, Peressigsäure, m-Chlorperbenzoesäure und "Chlorlauge" bzw. "Chlorwasser" ($Cl_2/H_2O$).

Als Verdünnungsmittel kommen bei der Durchführung des erfindungsgemäßen Verfahrens (b) alle üblicherweise für derartige Oxidationen verwendbaren organischen Lösungsmittel in Betracht. Vorzugsweise verwendbar sind Wasser, Eisessig, Chloroform oder Methylenchlorid.

Als Katalysatoren können beim erfindungsgemäßen Verfahren (b) alle üblicherweise für derartige Oxidationen verwendbaren Reaktionsbeschleuniger eingesetzt werden. Vorzugsweise verwendbar sind Ameisensäure, Schwefelsäure und Ammoniummolybdat. Bevorzugt wird ohne Katalysator gearbeitet.

Die Reaktionstemperaturen können bei dem erfindungsgemäßen Verfahren (b) in einem größeren Bereich variiert werden. Im allgemeinen arbeitet man bei Temperaturen zwischen -20 °C und +80 °C, vorzugsweise bei Temperaturen zwischen 0 °C und 50 °C. Das erfindungsgemäße Verfahren (b) wird im allgemeinen unter Normaldruck durchgeführt.

Bei der Durchführung des erfindungsgemäßen Verfahrens (b) werden auf 1 Mol der Verbindung der Formel (Ia) zwischen 1 und 5 Mol Oxidationsmittel eingesetzt. Die Umsetzung wird im allgemeinen in einem Verdünnungsmittel und gegebenenfalls in Gegenwart von Katalysatoren durchgeführt. Die Aufarbeitung geschieht nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die neuen Wirkstoffe der Formel (I) eignen sich zur selektiven Bekämpfung dikotyler Unkräuter in mono-und dikotylen Kulturen wie z. B. Weizen und Soja, insbesondere im Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zu Unkrautbekämpfung Verwendung finden, wobei Fertigformulierungen oder Tankmischungen möglich sind.

Für die Mischungen können bekannte Herbizide verwendet werden wie z. B.

N-(2-Benzthiazolyl)-N,N'-dimethylharnstoff, 3-(4-Isopropylphenyl)-1,1-dimethylharnstoff, 2-Chlor-N-{[(4-methoxy-6-methyl-1,3,5-triazin-2-yl)-amino]-carbonyl}-benzolsulfonamid, Ethyl-2-{[(4-chlor-6-methoxy-2-pyrimidyl)-aminocarbonyl]-aminosulfonyl}-benzoat, 2-Ethylamino-6-(1,1-dimethylethyl-amino)-4-methylthio-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 2-[4-(2,4-Dichlorphenoxy)-phenoxy]-propionsäuremethylester, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, 2-[5-Methyl-5-(1-methylethyl)-4-oxo-2-imidazolin-2-yl]-3-pyridincarbonsäure, 2-(1-Ethoxyamino-butyliden)-5-(2-ethylthiopropyl)-1,3-cyclohexandion, 2-[1-(Ethoxyimino)-butyl]-3-hydroxy-5-(tetrahydro-2H-thiopyran-3-yl)-2-cyclohexen-1-on, 2-(1-Alloxyaminobutyliden)-4-methoxycarbonyl-5,5-dimethylcyclohexan-1,3-dion-Natriumsalz, [(4-Amino-3,5-dichlor-6-fluor-2-pyridyl)oxy]-essigsäure und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie werden bevorzugt nach dem Auflaufen der Pflanzen eingesetzt.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

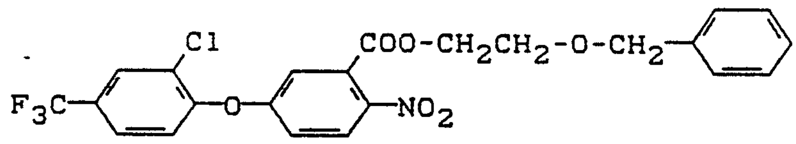

(Verfahren (a))

Eine Lösung von 7,6 g (0,02 Mol) 5-(2-Chlor-4-trifluormethyl-phenoxy)-2-nitro-benzoesäure-chlorid in 50 ml Methylenchlorid wird tropfenweise zu einer Mischung aus 3,0 g (0,02 Mol) 2-Benzyloxy-ethanol, 2 g Pyridin und 50 ml Methylenchlorid unter Rühren gegeben, wobei die Innentemperatur bei 0 °C bis +5 °C gehalten wird. Das Reaktionsgemisch wird dann 20 Stunden bei 20 °C gerührt, mit 1N-Salzsäure und mit Sodalösung gewaschen, mit Natriumsulfat getrocknet und filtriert. Vom Filtrat wird das Lösungsmittel im Wasserstrahlvakuum sorgfältig abdestilliert und der Rückstand durch Verreiben mit n-Hexan zur Kristallisation gebracht und das Produkt wird durch Absaugen isoliert.

Man erhält 5,7 g (58 % der Theorie) 5-(2-Chlor-4-trifluormethyl-phenoxy)-2-nitro-benzoesäure-(2-benzyloxy-ethyl)-ester in Form weißer Kristalle vom Schmelzpunkt 85 °C.

Die Verbindung des Beispiels (1) läßt sich auch gemäß Verfahren (a) folgendermaßen herstellen:

Eine Lösung von 121 g (1,20 Mol) Triethylamin in 100 ml Toluol wird innerhalb von ca. 2 Stunden zu einer Mischung aus 380 g (1,00 Mol) 5-(2-Chlor-4-trifluormethyl-phenoxy)-2-nitro-benzoesäure-chlorid, 160 g (1,05 Mol) 2-Benzyloxy-ethanol und 2 l Toluol bei 20 °C bis 40 °C unter Rühren gegeben. Das Reaktionsgemisch wird dann noch ca. 4 Stunden bei 30 °C bis 40 °C gerührt, anschließend zweimal mit kalter 1N-Salzsäure und zweimal mit kalter gesättigter Sodalösung gewaschen, mit Natriumsulfat getrocknet und filtriert. Vom Filtrat wird das Lösungsmittel im Wasserstrahlvakuum sorgfältig abdestilliert, der Rückstand durch Verreiben mit n-Hexan zur Kristallisation gebracht und das Produkt durch Absaugen isoliert.

Man erhält 436 g (88 % der Theorie) 5-(2-Chlor-4-trifluormethyl-phenoxy)-2-nitro-benzoesäure-(2-benzyloxy-ethyl)-ester in Form weißer Kristalle vom Schmelzpunkt 85 °C.

Beispiel 2

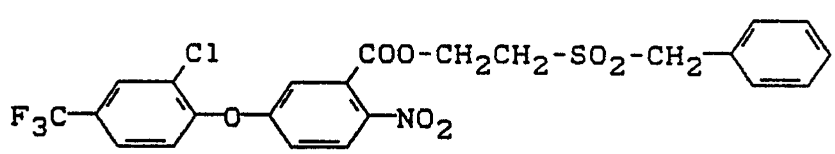

(Verfahren (b))

10 g einer 35 %igen wässrigen Lösung von Wasserstoffperoxid (0,10 Mol $H_2O_2$) werden tropfenweise bei 20 °C bis 40 °C zu einer Mischung aus 10,2 g (0,02 Mol) 5-(2-Chlor-4-trifluormethyl-phenoxy)-2-nitro-benzoesäure-(2-benzylthioethyl)-ester und 100 ml Eisessig unter Rühren gegeben. Das Reaktionsgemisch wird 3 Stunden bei 40 °C gerührt, mit Methylenchlorid auf etwa das doppelte Volumen verdünnt, mit wässriger Natriumhydrogensulfitlösung gewaschen, dann mit Wasser gewaschen, mit Natriumsulfat getrocknet und filtriert. Vom Filtrat wird das Lösungsmittel im Wasserstrahlvakuum sorgfältig abdestilliert.

Man erhält 6,8 g (63 % der Theorie) 5-(2-Chlor-4-trifluormethyl-phenoxy)-2-nitro-benzoesäure-(2-benzylsulfonyl-ethyl)-ester vom Schmelzpunkt 105 °C.

Analog Beispiel 1 und 2 bzw. analog Verfahren (a) und (b) können die in der nachfolgenden Tabelle 3 aufgeführten Verbindungen der Formel (I) hergestellt werden:

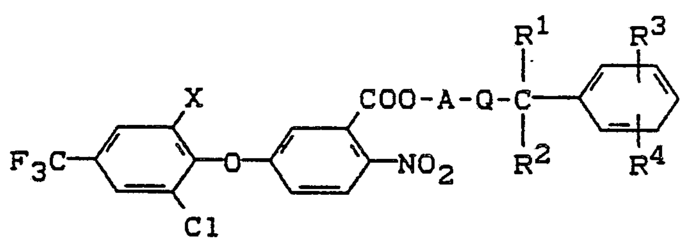

(I)

Tabelle 3

| Beisp.-Nr. | X | A | Q | $R^1$ | $R^2$ | $R^3$ | $R^4$ | Physikalische Konstante |
|---|---|---|---|---|---|---|---|---|
| 3 | Cl | $-CH_2CH_2-$ | S | H | H | H | H | $n_D^{20}$: 1,5782 |
| 4 | Cl | $-(CH_2)_3-$ | O | H | H | H | H | amorph |
| 5 | H | $-(CH_2)_3-$ | O | H | H | H | H | $n_D^{20}$: 1,5546 |
| 6 | Cl | $-CH_2CH_2-$ | O | H | H | H | H | $n_D^{20}$: 1,5580 |
| 7 | H | $-CH_2CH_2-$ | S | H | H | H | H | $n_D^{20}$: 1,5785 |
| 8 | H | $-CH_2CH_2-$ | O | H | H | 2-Cl | H | Fp. 87 °C |
| 9 | H | $-CH_2-C(CH_3)_2-CH_2-$ | O | H | H | H | H | $n_D^{20}$: 1,5408 |
| 10 | Cl | $-CH_2CH_2-$ | O | H | H | 4-F | H | |
| 11 | H | $-CH_2CH_2-$ | O | H | H | 4-F | H | |
| 12 | Cl | $-CH_2CH_2-$ | O | H | H | 2-Cl | 6-Cl | |
| 13 | H | $-CH_2CH_2-$ | O | H | H | 2-Cl | 6-Cl | |

<u>Tabelle 3</u> - Fortsetzung

| Beisp.-Nr. | X | A | Q | $R^1$ | $R^2$ | $R^3$ | $R^4$ | Physikalische Konstante |
|---|---|---|---|---|---|---|---|---|
| 14 | Cl | $-CH(CH_3)-CH_2-$ | O | H | H | H | H | |
| 15 | H | $-CH(CH_3)-CH_2-$ | O | H | H | H | H | |
| 16 | Cl | $-CH_2CH_2-$ | O | H | H | 2-F | H | |
| 17 | H | $-CH_2CH_2-$ | O | H | H | 2-F | H | |
| 18 | Cl | $-CH_2CH_2-$ | O | H | $CH_3$ | H | H | |
| 19 | H | $-CH_2CH_2-$ | O | H | $CH_3$ | H | H | |
| 20 | Cl | $-CH_2CH_2-$ | S | H | H | 4-Cl | H | |
| 21 | H | $-CH_2CH_2-$ | S | H | H | $4-OCH_3$ | H | |
| 22 | Cl | $-CH_2CH_2-$ | $SO_2$ | H | H | H | H | |

Anwendungsbeispiel

In dem folgenden Anwendungsbeispiel wird die nachstehend aufgeführte Verbindung als Vergleichssubstanz herangezogen:

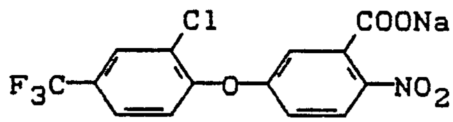

(A)

Natriumsalz der 3-(2-Chlor-4-trifluormethyl-phenoxy)-6-nitro-benzoesäure (bekannt aus US-P 4 063 929).

Beispiel A

Post-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigt z.B. der Wirkstoff gemäß Beispiel (1) bei der Bekämpfung von dikotylen Unkräutern wie z. B. Chenopodium, Sida und Viola in Weizen und Soja eine bessere Wirksamkeit als die Vergleichssubstanz (A).

## Ansprüche

1. Phenoxybenzoesäureester der Formel (I)

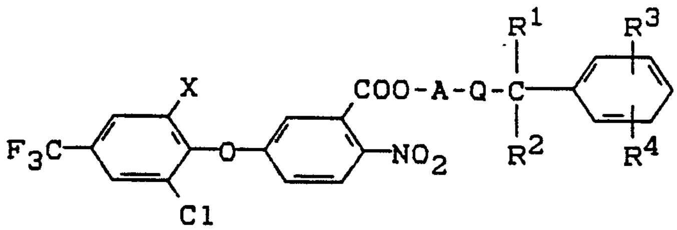

(I)

in welcher

X für Wasserstoff oder Halogen steht,
A für gegebenenfalls verzweigtes $C_2$-$C_6$-Alkandiyl steht,
Q für Sauerstoff, Schwefel, Sulfinyl (SO) oder Sulfonyl ($SO_2$) steht,
$R^1$ für Wasserstoff oder Methyl steht,

$R^2$ für Wasserstoff oder $C_1$-$C_4$-Alkyl steht und

$R^3$ und $R^4$ gleich oder verschieden sind und für Wasserstoff, Halogen, Cyano, Nitro, $C_1$-$C_4$-Alkyl, Halogen-$C_1$-$C_2$-alkyl, $C_1$-$C_4$-Alkoxy, Halogen-$C_1$-$C_2$-alkoxy und $C_1$-$C_2$-Alkoxycarbonyl stehen.

2. Phenoxybenzoesäureester der Formel (I) gemäß Anspruch 1,
in welcher

X für Wasserstoff, Fluor oder Chlor steht,

A für gegebenenfalls verzweigtes $C_2$-$C_5$-Alkandiyl steht,

Q für Sauerstoff, Schwefel, Sulfinyl oder Sulfonyl steht,

$R^1$ für Wasserstoff steht,

$R^2$ für Wasserstoff oder Methyl steht und

$R^3$ und $R^4$ gleich oder verschieden sind und für Wasserstoff, Fluor, Chlor, Cyano, Nitro, Methyl, Trifluormethyl, Methoxy, Trifluormethoxy oder Methoxycarbonyl stehen.

3. Phenoxybenzoesäureester der Formel (I) gemäß Anspruch 1,
in welcher

X für Wasserstoff oder Chlor steht,

A für gegebenenfalls verzweigtes $C_2$-$C_5$-Alkandiyl steht,

Q für Sauerstoff, Schwefel oder Sulfonyl steht,

$R^1$ für Wasserstoff steht,

$R^2$ für Wasserstoff oder Methyl steht und

$R^3$ und $R^4$ gleich oder verschieden sind und für Wasserstoff, Fluor, Chlor oder Methoxy stehen.

4. Verfahren zur Herstellung von Phenoxybenzoesäureestern der Formel (I)

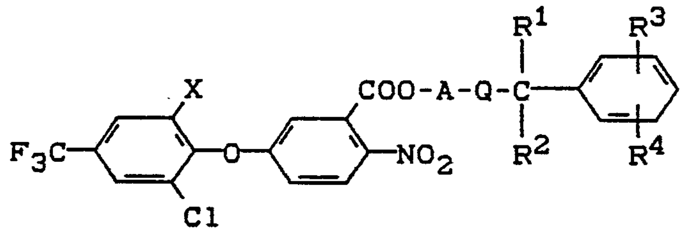

(I)

in welcher

X für Wasserstoff oder Halogen steht,

A für gegebenenfalls verzweigtes $C_2$-$C_6$-Alkandiyl steht,

Q für Sauerstoff, Schwefel, Sulfinyl (SO) oder Sulfonyl ($SO_2$) steht,

$R^1$ für Wasserstoff oder Methyl steht,

$R^2$ für Wasserstoff oder $C_1$-$C_4$-Alkyl steht und

$R^3$und $R^4$ gleich oder verschieden sind und für Wasserstoff, Halogen, Cyano, Nitro, $C_1$-$C_4$-Alkyl, Halogen-$C_1$-$C_2$-alkyl, $C_1$-$C_4$-Alkoxy, Halogen-$C_1$-$C_2$-alkoxy und $C_1$-$C_2$-Alkoxycarbonyl stehen,

dadurch gekennzeichnet, daß man

(a) Phenoxybenzoesäurechloride der Formel (II)

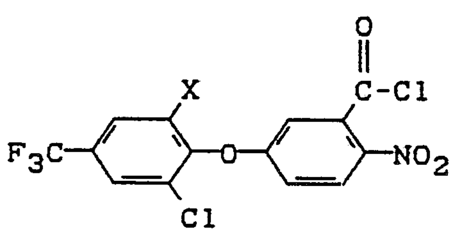

(II)

in welcher

X die oben angegebene Bedeutung hat,

mit Hydroxyverbindungen der Formel (III)

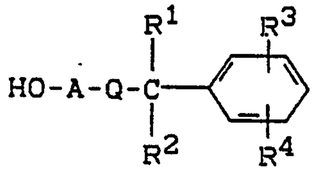

(III)

in welcher

A, Q, $R^1$, $R^2$, $R^3$ und $R^4$ die oben angegebene Bedeutung haben,

gegebenenfalls in Gegenwart eines Säurebindemittels sowie gegebenenfalls in Gegenwart eines Verdünnungsmittels umsetzt,

oder daß man außerdem für den Fall, daß Verbindungen der Formel (I) hergestellt werden sollen, in denen Q für Sulfinyl oder Sulfonyl steht,

(b) die Verbindungen der Formel (Ia)

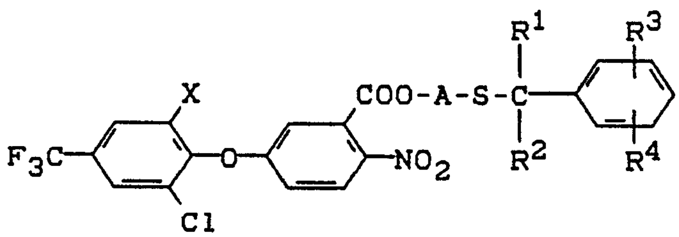

(Ia)

in welcher

A, X, $R^1$, $R^2$, $R^3$ und $R^4$ die oben angegebenen Bedeutungen haben,

mit Oxidationsmitteln, gegebenenfalls in Gegenwart eines Katalysators und gegebenenfalls in Gegenwart eines Verdünnungsmittels umsetzt.

5. Herbizide Mittel, gekennzeichnet durch einen Gehalt an mindestens einem Phenoxbenzoesäureester der Formel (I) gemäß den Ansprüchen 1 bis 4.

6. Verfahren zur Bekämpfung von Unkräutern, dadurch gekennzeichnet, daß man Phenoxybenzoesäureester der Formel (I) gemäß den Ansprüchen 1 bis 4 auf die Unkräuter und/oder ihren Lebensraum einwirken läßt.

7. Verwendung von Phenoxybenzoesäureestern der Formel (I) gemäß den Ansprüchen 1 bis 4 zur Bekämpfung von Unkräutern.

8. Verfahren zur Herstellung von herbiziden Mitteln, dadurch gekennzeichnet, daß man Phenoxybenzoesäureester der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 87111538.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB - A - 2 068 948 (MOBIL OIL)<br>* Seite 3, Zeile 23 - Seite 5, Zeile 4, insbesondere Seite 4, Zeile 12; Ansprüche 1, 25,26 *<br>-- | 1,4-8 | C 07 C 79/46<br>C 07 C 149/273<br>C 07 C 147/00<br>A 01 N 37/48<br>A 01 N 41/10 |
| D,A | EP - A1 - 0 092 112 (NIHON TOKUSHU NOYAKU SEIZO)<br>* Ansprüche 1,3-5 *<br>-- | 1,4-8 | |
| A | GB - A - 2 137 988 (ICI)<br>* Ansprüche 1,9-12; Tabelle 1 * | 1,4-8 | |
| D,A | & EP-A1-0 122 037<br>---- | | |

| RECHERCHIERTE SACHGEBIETE (Int. Cl.4) |
|---|
| C 07 C 79/ |
| C 07 C 149/ |
| C 07 C 147/ |

Der vorliegende Recherchenbericht wurde für alle Patentansprüche erstellt.

| Recherchenort | Abschlußdatum der Recherche | Prüfer |
|---|---|---|
| WIEN | 09-11-1987 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument